# EUROPÄISCHE PATENTSCHRIFT

(11) **EP 1 137 443 B1**
(45) Veröffentlichungstag und Bekanntmachung des Hinweises auf die Patenterteilung: **12.03.2003**
(21) Anmeldenummer: 99963379.5
(22) Anmeldetag: 03.12.1999
(51) Int. Cl.: A61K 49/00, A61K 49/06

(54) **MRT-KONTRASTMITTEL**
MAGNETIC RESONANCE TOMOGRAPHY CONTRAST AGENT
SUBSTANCE DE CONTRASTE DESTINEE A LA TOMOGRAPHIE PAR RESONANCE MAGNETIQUE (MRT)

(30) Priorität: 11.12.1998 DE 19857345
(43) Veröffentlichungstag der Anmeldung: 04.10.2001
(73) Patentinhaber: Sanochemia Diagnostics Deutschland GmbH, 41460 Neuss (DE)
(72) Erfinder: HIEBL, Wilfried, D-89257 Illertissen (DE); FRANK, Artur, 89340 Leipheim (DE)
(74) Vertreter: Sternagel, Fleischer, Godemeyer & Partner Patentanwälte
(86) Internationale Anmeldenummer: EP9909459
(87) Internationale Veröffentlichungsnummer: WO00035489

(56) Entgegenhaltungen:
- EP-A- 0 564 307
- WO-A-94/27499
- US-A- 4 615 879
- US-A- 4 927 624

## Beschreibung

Die Erfindung bezieht sich auf ein MRT-Kontrastmittel mit einer Viskosität von 500 bis 2500 cP, welches eine Suspension von Kaolin gegebenenfalls in Verbindung mit Bentonit in Citratpuffer umfaßt, sowie ein Verfahren zur Herstellung derartiger MRT-Kontrastmittel.

Die Magnetresonanztomographie, auch bezeichnet als MR-Tomographie, MRT, Kernspinresonanztomographie oder NMR-Tomographie (im Englischen "nuclear spin tomography", "magnetic resonance imaging" oder "MRI"), ist eine medizinische Abbildungstechnik insbesondere für Gewebe, v.a. Weichteilgewebe, bei der im Gegensatz zur konventionellen Röntgendiagnostik bzw. Computertomographie keine ionisierende Strahlung eingesetzt wird. Vielmehr wird bei der Magnetresonanztomographie die Energie gemessen, die unter Einfluß eines von außen angelegten starken Magnetfeldes bei Relaxation der durch einen kurzen Hochfre-quenzimpuls angeregten Kernspins aus dem Körper in Form von elektromagnetischen Wellen austritt.

Typische Einsatzgebiete für die Magnetresonanztomographie sind v.a. Erkrankungen des Nervengewebes, wie Hirn- und Rükkenmark, insbesondere bei Multipler Sklerose, Schädigungen von Bandscheiben, Gelenken und Muskeln.

Darüber hinaus wird die Magnetresonanztomographie auch für die Abdominaldiagnostik eingesetzt. In diesem letztgenannten Zusammenhang stellt jedoch die Tatsache, daß die Qualität und insbesondere der Kontrast der erhaltenen Abbildungen z.T. in beträchtlichem Ausmaß vom Magen- und Darminhalt, der je nach Patient stark variieren kann, beeinflußt wird, ein Problem dar. In Kenntnis dieser Problematik wurde früh erkannt, daß die Genauigkeit der Darstellung durch Einsatz von Kontrastmitteln zur Unterscheidung des Magens und des Darms von umgebendem Gewebe stark verbessert werden konnte.

Diverse Kontrastmitteltypen sind für einen Einsatz bei der Magnetresonanztomographie zur Abdominaldiagnostik vorgeschlagen worden. Hierzu zählen u.a. paramagnetische und supraparamagnetische Kontrastmittel, wie z.B. Eisen(III)-Verbindungen, wie Eisen(III)-chlorid oder Eisen(III)-ammoniumcitrat, Gadolinium-Verbindungen, wie Gadolinium-DTPA, und Mangansalze, die gegebenenfalls zur Verringerung ihrer Toxizität in fester partikulärer Form als Suspensionen eingesetzt werden, sowie Mineralöle und Perfluorkohlenstoffverbindungen.

Als preiswerte und nicht-toxische Alternative zu den genannten MRT-Kontrastmitteln wurde in dem US-Patent 4,927,624 die Verwendung von Suspensionen von Tonmineralen in Wasser als oral oder rektal verabreichbare MRT-Kontrastmittel für eine Abbildung des Gastrointestinaltrakts vorgeschlagen. Als beispielhafte Tonminerale werden in dieser Patentschrift Kaolin, die Montmorillonite, z.B. Bentonit, Hektorit, Beidellit, Sauconit und Nontronit erwähnt.

Insbesondere bei einer oralen Verabreichung ist die Viskosität der verabreichten Kontrastmittelsuspension von zentraler Bedeutung für die Akzeptanz durch den Patienten. Da die von dem Patienten aufzunehmenden Mengen an Suspension beträchtlich sind, sollte sie eine ausreichend niedrige Viskosität aufweisen, um die orale Aufnahme für den Patienten erträglich zu machen. Andererseits darf die Viskosität nicht zu gering sein, da sonst der Beschlag, d.h. die Benetzung der Magen- und Darmwände nicht ausreichend gut ist, was auch den Kontrast der Aufnahme beeinträchtigen würde. Im Rahmen der Untersuchungen in Zusammenhang mit der hier erläuterten Erfindung ist ermittelt worden, daß eine Viskosität einer derartigen MRT-Kontrastmittel-suspension im Bereich von 500 bis 2500 cP vom Patienten gut akzeptiert wird und auch einen ausreichenden Beschlag sicherstellt.

Es sind gegenwärtig viele unterschiedliche Tonminerale, wie Kaolin und Bentonit, in einer Vielzahl unterschiedlicher Qualitäten und z.T. auch in unterschiedlichen Kristallformen im Handel erhältlich. Die einfache Suspendierung handelsüblicher Tonminerale, wie Bentonit, zu einer u.a. für einen ausreichenden Beschlag erforderlichen Konzentration in Wasser, wie sie in dem US-Patent 4,927,624 beschrieben wird, führt in aller Regel zu pasten- oder zementartigen Präparationen sehr hoher Viskosität, die für eine Verabreichung an Patienten unzumutbar sind. Darüber hinaus ist bei hochviskosen Präparationen, deren Viskosität 2500 cP überschreitet, die Passagezeit durch den Gastrointestinaltrakt bis zu dem Zielort im Magen und/oder Darm deutlich verlängert. Je nach Viskosität kann die Passagezeit von einer normalen Zeitdauer von 30 bis 45 min auf 1,5 h oder mehr ansteigen, was für Patient und Arzt gleichermaßen unerwünscht ist.

Zur Verringerung der Viskosität von Kaolinsuspensionen ist im Stand der Technik die Verwendung von Laurylsulfat bekannt. Jedoch schäumen derartige Suspensionen stark, was den Kontrast der MRT-Aufnahme bedeutend verschlechtern kann.

Die der Erfindung zugrundeliegende Aufgabe bestand dementsprechend darin, alternative MRT-Kontrastmittel auf Basis von Tonmineralen und insbesondere von Kaolin, bereitzustellen, die eine für einen guten Beschlag und einen guten Kontrast ausreichende Konzentration an Tonmineral aufweisen, gleichzeitig eine ausreichend niedrige Viskosität aufweisen, um von einem Patienten ohne zu großen Widerwillen auf oralem Wege aufgenommen zu werden, darüber hinaus nicht schäumen, wodurch stets ein guter T₁- und T₂-Kontrast gewährleistet wird, und gute Langzeitstabilität aufweisen.

Diese Aufgabe wird von den in den Ansprüchen definierten MRT-Kontrastmitteln gelöst.

Wesentlich für die Lösung der erfindungsgemäßen Aufgabe ist die Einstellung der gewünschten Viskosität mit einem geeigneten Kaolin in dem gewählten Konzentrationsbereich. Als ganz besonders vorteilhaft hat sich sogenanntes Kaolin leicht bewährt, das im folgenden noch näher erörtert wird. Darüberhinaus hat sich herausgestellt, daß die Verwendung eines Citronensäure/Citrat-Puffersystems und somit die Einstellung eines pH-Wertes im Bereich 6-8 besonders vorteilhaft ist.

Das erfindungsgemäße MRT-Kontrastmittel, das eine Viskosität in dem akzeptablen Viskositätsbereich von 500 bis 2500 cP aufweist, ist dadurch gekennzeichnet, daß es eine Suspension von 25-50 Gew.-% Kaolin in 6-10 mmol/l Citratpuffer, pH 6-8, umfaßt.

Ein von Patienten in der Regel bevorzugter Viskositätsbereich liegt zwischen 500 und 1800 cP, und als besonders akzeptabel werden erfindungsgemäße MRT-Kontrastmittelsuspensionen empfunden, deren Viskosität 1500 cP nicht überschreitet. Die in den vorliegenden Unterlagen angegebenen Viskositäten wurden mit einem Brookfield-Viskosimeter DV II+ mit Spindel 3, 3 x 3 Upm und 3 x 6 Upm (= 3 min bei 3 Upm und 3 min bei 6 Upm) bei 20°C gemessen.

Die Wahl des Citratpuffersystems mit einer Molarität im Bereich von 6-10 mmol/l und einem pH zwischen 6 und 8 ist erfindungswesentlich. Vergleichsversuche mit Phosphatpuffern von in etwa gleicher Molarität und mit einem pH zwischen 6 und 8 ergaben zu dünnflüssige Suspensionen (Viskosität < 500 cP), die keinen ausreichenden Beschlag und auch keinen ausreichenden Kontrast lieferten. In bevorzugten Ausführungsformen liegt der pH des Citratpuffers zwischen 6,5 und 7,5 und die Citratpufferkonzentration zwischen 7,5 und 8,2 mmol/l.

Das erfindungsgemäße MRT-Kontrastmittel umfaßt 25-50 (28-50) Gew.-% Kaolin und in bevorzugten Ausführungsformen 25-40 Gew.-% Kaolin.. Unterhalb von 25 Gew.-% Kaolin sind der Beschlag und auch der Kontrast unzureichend.

Das für die erfindungsgemäße MRT-Kontrastmittel einsetzbare Kaolin muß sorgfältig ausgewählt werden. Im Rahmen der Erfindung findet insbesondere Kaolin leicht mit einem geringen Quellvermögen Anwendung. Allerdings muß das Kaolin leicht den Quellfähigkeitstest der Europäischen und Deutschen Pharmacopoieia bestehen, bei welchem bei Verreiben von 2 g Kaolin leicht mit 2 ml Wasser die entstehende Mischung nicht auseinander fließen darf. Das Kaolin leicht entspricht vollständig der Definition für "Light Kaolin" der British Pharmacopoieia, HMSO Publications Center, London, 1993, weist also pharmazeutische Qualität zur inneren Anwendung, auf.

Ein für die erfindungsgemäßen MRT-Kontrastmittel besonders geeignetes Kaolin wird unter der Bezeichnung "Kaolin light" von der Firma Richard Baker Harrison Ltd., Ilford, Vereinigtes Königreich, vertrieben. Dieses Kaolin weist eine ursprüngliche Korngröße zwischen 7 und 9 µm auf und kann ohne jegliche Vorbehandlung zur Herstellung der erfindungsgemäßen MRT-Kontrast-mittel eingesetzt werden.

In einer ganz besonders bevorzugten Ausführungsform umfaßt das erfindungsgemäße MRT-Kontrastmittel zusätzlich Bentonit und insbesondere in einer Konzentration von 4-5 Gew.-%. Bei Zusatz von Bentonit in der vorstehend angegebenen Konzentration enthält das erfindungsgemäße MRT-Kontrastmittel Kaolin leicht bevorzugt in einer Konzentration von 25-42 Gew.-%. Oberhalb von 42-43 Gew.-% Kaolin leicht kann bei einem derartigen MRT-Kontrastmittel in Abhängigkeit von den verwendeten Tonmineralqualitäten häufig eine deutliche Viskositätserhöhung auftreten.

Ganz analog zu dem Kaolin-Bestandteil muß der Bentonit für einen Einsatz in dem erfindungsgemäßen MRT-Kontrastmittel sorgfältig ausgewählt werden. Neben pharmazeutischer Qualität für die innere Anwendung darf der Bentonit nur ein sehr niedriges Quellvermögen, d.h. ein Quellvermögen im Bereich von ungefähr 16 bis maximal 20 ml pro 2 g aufweisen.

Eine besonders geeignete Spezial-Bentonit-Qualität wird von der Firma Süd-Chemie AG, Moosburg, Deutschland, unter der Bezeichnung EX75 vertrieben. Dieser Bentonit weist eine Quellfähigkeit von 16 bis 20 ml pro 2 g auf, was deutlich unter dem Wert von 22 ml pro 2 g liegt, der von den Arzneibüchern vorgeschrieben wird. Seine ursprüngliche Korngröße beträgt 12 - 17 µm. Dieser Bentonit kann ohne jegliche Vorbehandlung zur Herstellung der erfindungsgemäßen MRT-Kontrastmittel eingesetzt werden.

In einer weiteren bevorzugten Ausführungsform des erfindungsgemäßen MRT-Kontrastmittels werden das Kaolin leicht oder das Kaolin leicht und der Bentonit oder der Bentonit allein mit einer mittleren Korngröße von 2,5-5 µm eingesetzt. Hierzu können geeignete Kaolin- und Bentonit-Qualitäten gegebenenfalls vor der Herstellung des Kontrastmittels auf eine mittlere Korngröße in dem angegebenen Korngrößenbereich mikronisiert werden. Werden Kaolin leicht und Bentonit mit einer mittleren Korngröße von weniger als ca. 2 µm eingesetzt, ist eine starke Erhöhung der Viskosität festzustellen, so daß derartige Kaolin- und Bentonit-Ausgangsminerale für die erfindungsgemäßen MRT-Kontrast-mittel ungeeignet sind.

Die Mikronisierung auf eine Korngröße in dem angegebenen Korngrößenbereich von 2,5-5 µm führt zu einer geringfügigen, aber dennoch in der Regel erwünschten Viskositätserniedrigung, zu einer Kontrastverbesserung aufgrund der Erhöhung der Partikeloberfläche und darüber hinaus zu einer Geschmacksverbesserung, da der sandartige Geschmack der MRT-Kontrastmittel gemildert wird.

Darüber hinaus können die erfindungsgemäßen MRT-Kontrastmittel übliche Zusatz- und Hilfsstoffe, wie Geschmacksstoffe, z.B. Cyclamat, Saccharin, Sorbit, Fruchtaromen, oder Konservierungsmittel, wie Parabene, in üblicherweise eingesetzten Mengen enthalten. Diese Zusatz- und Hilfsstoffe haben keinerlei Einfluß auf das Fließverhalten und die Viskosität der erfindungsgemäßen MRT-Kontrastmittel.

Die erfindungsgemäßen MRT-Kontrastmittel weisen sämtlich diverse Vorteile auf, nämlich gute Trinkbarkeit aufgrund ausreichend niedriger Viskosität, einen guten T₁- und T₂-Kontrast, gute Beschlagseigenschaften und Vermeidung von Schaumbildung, sowie eine hervorragende Langzeitstabilität der Suspension von mindestens 2 bis 3 Jahren. Sie sind sowohl für eine orale als auch rektale Verabreichung geeignet.

Darüber hinaus bezieht sich die Erfindung auf Verfahren zur Herstellung der erfindungsgemäßen MRT-Kontrastmittel.

Ein Verfahren zur Herstelllung eines erfindungsgemäßen MRT-Kontrastmittels auf Basis von Kaolin allein umfaßt die folgenden Schritte:
- Lösen von Citronensäure und eines Citratsalzes und gegebenenfalls weiterer Hilfsstoffe in 75-85% des benötigten Wassers unter Rühren zur Bildung einer Citrat-gepufferten Lösung mit einem pH zwischen 6 und 8,
- portionsweises Zusetzen des Kaolin leicht unter Rühren, jeweils gefolgt von mehrminütigem Nachrühren zur Suspendierung des Kaolin leicht,
- Zusetzen des restlichen Wassers,
- abschließendes mehrstündiges Rühren.

Ein Verfahren zur Herstellung eines MRT-Kontrastmittels, das zusätzlich Bentonit enthält, ist durch die folgenden Schritte gekennzeichnet:
- Lösen von Citronensäure und eines Citratsalzes und gegebenenfalls weiterer Hilfsstoffe in 75-85% des benötigten Wassers unter Rühren zur Bildung einer Citrat-gepufferten Lösung mit einem pH zwischen 6 und 8,
- portionsweises Zusetzen von 70 - 85% des Kaolin leicht unter Rühren, jeweils gefolgt von mehrminütigem Nachrühren zur Suspendierung des Kaolin leicht,
- Vermischen der übrigen 30 - 15% des Kaolin leicht mit dem Bentonit in trockenem Zustand zu einer homogenen Mischung,
- portionsweises Zugeben der homogenen Mischung zu der bereits hergestellten Kaolinsuspension unter Rühren, jeweils gefolgt von mehrminütigem Nachrühren zur Suspendierung der homogenen Mischung,
- Zusetzen des restlichen Wassers,
- abschließendes mehrstündiges Rühren.

Die in dem vorstehend beschriebenen Herstellungsverfahren für ein Kaolin und Bentonit enthaltendes MRT-Kontrastmittel erläuterte Abfolge der Komponentenzugabe kann in Abhängigkeit von dem verwendeten Kaolin leicht und Bentonit für die Erzielung der gewünschten Viskosität des MRT-Kontrastmittels von Bedeutung sein.

Die Suspendierungsschritte können gegebenenfalls bei erhöhter Temperatur, z.B. zwischen 50 und 60°C durchgeführt werden.

Das Nachrühren nach jeder Einzelzugabe von Kaolin leicht oder homogener Mischung erfolgt vorzugsweise für mindestens 10 min und insbesondere für 10 bis 15 min. Der abschließende Rührvorgang erfolgt vorzugsweise für mindestens 2 h.

Die Rührgeschwindigkeit muß dabei ggf. individuell gesteuert werden, um einen irreversiblen Viskositätsanstieg aufgrund zu hoher Rührgeschwindigkeit zu vermeiden. Im vorliegenden Zusammenhang hat sich eine Rührgeschwindigkeit bei der Suspendierung des Kaolin leicht und/oder der homogenen Mischung sowie bei dem abschließenden Rührvorgang von 350 ± 50 Upm bewährt.

Gegebenenfalls können das Kaolin leicht und/oder der Bentonit vorab einer Mikronisierungsbehandlung auf eine geringere Korngröße unterworfen werden. Wie bereits erläutert, führt eine Mikronisierung u.a. zur Erhöhung der Partikeloberfläche der Tonminerale und aufgrunddessen zu einem besseren Kontrast der MRT-Abbildung.

In einer bevorzugten Ausführungsform des erfindungsgemäßen Verfahrens läßt man das MRT-Kontrastmittel nach dem abschließenden Rühren zusätzlich lagern, wodurch dessen Viskosität teilweise bedeutend, z.B. von Werten über 2500 cP auf Werte deutlich unter 2500 cP, verringert werden kann. Diese Lagerung, die bei Raumtemperatur oder leicht erhöhter Temperatur und bei normaler Luftfeuchtigkeit erfolgen kann, erfolgt in der Regel für mindestens 24 h. Die Viskositätsverringerung ist üblicherweise innerhalb von zwei bis fünf Tagen abgeschlossen.

Im Folgenden wird die Erfindung anhand eines Beispiels, das die Herstellung zweier erfindungsgemäßer MRT-Kontrastmittel beschreibt, näher erläutert.

### Beispiel

Gemäß dem nachfolgend beschriebenen Herstellungsverfahren wurden zwei MRT-Kontrastmittel mit der folgenden Zusammensetzung hergestellt:

| | MRT-Kontrastmittel mit 40% Kaolin | MRT-Kontrastmittel mit 30% Kaolin |
|---|---|---|
| Kaolin light | 432,0 g | 323,5 g |
| Bentonit EX75 | 49,5 g | 49,5 g |
| Methylparaben | 0,54 g | 0,54 g |
| Propylparaben | 0,243 g | 0,243 g |
| Natriumcitrat-dihydrat | 0,189 g | 0,189 g |
| Citronensäure | 1,08 g | 1,08 g |
| Natriumsaccharin | 0,54 g | 0,54 g |
| Bananenaroma | 3,24 g | 3,24 g |
| Wasser | 592,668 g | 701,168 g |
| Dichte | 1,369 g/cm³ | 1,234 g/cm³ |

Der pH der MRT-Kontrastmittel lag zwischen 6,5 und 7,5.

Vor Beginn des Herstellungsverfahrens wurden das Kaolin, "Kaolin light" von Richard Baker Harrison Ltd., Ilford, Vereinigtes Königreich, auf eine mittlere Korngröße von ca. 3,6 µm und der Bentonit, Bentonit EX75 von Süd-Chemie AG, Moosburg, Deutschland, auf eine mittlere Korngröße von ca. 4,2 µm mikronisiert.

In einem Behälter werden ungefähr 80% der benötigten Menge an sterilisiertem, bevorzugt entionisiertem, sterilisiertem und pyrogenfrei gemachtem Wasser vorgelegt und auf 55°C erwärmt. Anschließend werden das Methyl-4-hydroxybenzoat (Methyl-paraben), das Propyl-4-hydroxybenzoat (Propylparaben), das Natriumcitrat-dihydrat, das Natriumsaccharin als Dihydrat und die Citronensäure, wasserfrei, zugegeben. Es wird bei 350 ± 50 Upm solange gerührt, bis alle Substanzen vollständig gelöst sind.

80% der Gesamtmenge an Kaolin werden der Lösung portionsweise und unter Rühren (350 ± 50 Upm) zugesetzt. Nach jeder Einzelzugabe wird jeweils ca. 10 min nachgerührt (350 ± 50 Upm).

Die verbleibende Restmenge an Kaolin und die gesamte Menge an Bentonit werden-in einem separaten Behälter im trockenen Zustand homogen vermischt und anschließend ebenfalls portionsweise unter Rühren (350 ± 50 Upm) dem Ansatz zugesetzt. Nach jeder Einzelzugabe wird jeweils ca. 10 min nachgerührt (350 ± 50 Upm).

Nach Zugabe des Bananenaromas wird mit der Restmenge an Wasser auf das Sollgewicht des Ansatzes aufgefüllt.

Dieser Ansatz wird bis zum Vorliegen einer homogenen und klumpenfreien Suspension abschließend mindestens 2 h weitergerührt (350 ± 50 Upm).

Danach läßt man den Ansatz bis zu drei Tage lagern, wodurch die Viskosität der MRT-Kontrastmittel auf deutlich unter 2500 cP abfällt.

## Patentansprüche

1. MRT-Kontrastmittel mit einer Viskosität von 500 bis 2500 cP, umfassend eine Suspension von 25-50 Gew.-% Kaolin in 6-10 mmol/l Citratpuffer, pH 6-8.

2. MRT-Kontrastmittel nach Anspruch 1, **dadurch gekennzeichnet, daß** das Kaolin in einem 7,5-8,2 mmol/l Citratpuffer, pH 6-8 suspendiert ist.

3. MRT-Kontrastmittel nach Anspruch 1 oder Anspruch 2, **dadurch gekennzeichnet, daß** der pH des Citratpuffers zwischen 6,5 und 7,5 liegt.

4. MRT-Kontrastmittel nach einem der Ansprüche 1 bis 3, **dadurch gekennzeichnet, daß** es 25-40 Gew.-% Kaolin enthält.

5. MRT-Kontrastmittel nach einem der vorangegangenen Ansprüche, **dadurch gekennzeichnet, daß** es darüber hinaus Bentonit enthält.

6. MRT-Kontrastmittel nach Anspruch 5, **dadurch gekennzeichnet, daß** es 25-40 Gew.-% Kaolin und 4-5 Gew.-% Bentonit enthält.

7. MRT-Kontrastmittel nach einem der vorangegangenen Ansprüche, **dadurch gekennzeichnet, daß** das Kaolin und gegebenenfalls auch der Bentonit eine Korngröße von 2,5-5 µm aufweisen.

8. Verfahren zur Herstellung eines MRT-Kontrastmittels nach einem der Ansprüche 1 bis 4, **dadurch gekennzeichnet, daß** es die folgenden Schritte umfaßt:
- Lösen von Citronensäure und eines Citratsalzes und gegebenenfalls weiterer Hilfsstoffe in 75-85% des benötigten Wassers unter Rühren zur Bildung einer Citrat-gepufferten Lösung mit einem pH zwischen 6 und 8,
- portionsweises Zusetzen des Kaolins unter Rühren, jeweils gefolgt von mehrminütigem Nachrühren zur Suspendierung des Kaolins,
- Zusetzen des restlichen Wassers,
- abschließendes mehrstündiges Rühren.

9. Verfahren zur Herstellung eines MRT-Kontrastmittels nach Anspruch 5 oder 6, **dadurch gekennzeichnet, daß** es die folgenden Schritte umfaßt:
- Lösen von Citronensäure und eines Citratsalzes und gegebenenfalls weiterer Hilfsstoffe in 75-85% des benötigten Wassers unter Rühren zur Bildung einer Citrat-gepufferten Lösung mit einem pH zwischen 6 und 8,
- portionsweises Zusetzen von 70 - 85% des Kaolins unter Rühren, jeweils gefolgt von mehrminütigem Nachrühren zur Suspendierung des Kaolins,
- Vermischen der übrigen 30 - 15% des Kaolins mit dem Bentonit in trockenem Zustand zu einer homogenen Mischung,
- portionsweises Zugeben der homogenen Mischung zu der bereits hergestellten Kaolinsuspension unter Rühren, jeweils gefolgt von mehrminütigem Nachrühren zur Suspendierung der homogenen Mischung,
- Zusetzen des restlichen Wassers,
- abschließendes mehrstündiges Rühren.

10. Verfahren nach einem der Ansprüche 8 oder 9, **dadurch gekennzeichnet, daß** das Rühren zur Suspendierung des Kaolin leicht und/oder der homogenen Mischung bei 350 ± 50 Upm erfolgt.

11. Verfahren nach Anspruch 10, **dadurch gekennzeichnet, daß** das Nachrühren nach jeder Einzelzugabe von Kaolin oder homogener Mischung für 10 bis 15 min erfolgt.

12. Verfahren nach einem der Ansprüche 8 bis 11, **dadurch gekennzeichnet, daß** das abschließende mehrstündige Rühren bei 350 ± 50 Upm für mindestens 2 h erfolgt.

13. Verfahren nach einem der Ansprüche 8 bis 12, **dadurch gekennzeichnet, daß** das Kaolin und gegebenenfalls der Bentonit vorab einer Mikronisierungsbehandlung auf eine geringere Korngröße unterworfen werden.

14. Verfahren nach einem der Ansprüche 8 bis 13, **dadurch gekennzeichnet, daß** man das MRT-Kontrastmittel nach dem abschließenden Rühren zusätzlich lagern läßt.

15. Verfahren nach Anspruch 14, **dadurch gekennzeichnet, daß** die Lagerung für mindestens 24 h erfolgt.

16. Verfahren nach Anspruch 15, **dadurch gekennzeichnet, daß** die Lagerung für 2 bis 5 Tage erfolgt.

## Claims

1. An MRI contrast agent with a viscosity of from 500 to 2 500 cP, comprising a suspension of 25-50% by weight of kaolin in 6-10 mmol/l citrate buffer, pH 6-8.

2. The MRI contrast agent according to claim 1, **characterised in that** the kaolin is suspended in a 7.5-8.2 mmol/l citrate buffer, pH 6-8.

3. The MRI contrast agent according to claim 1 or claim 2, **characterised in that** the pH of the citrate buffer is between 6.5 and 7.5.

4. The MRI contrast agent according to one of claims 1 to 3, **characterised in that** it contains 25-40% by weight of kaolin.

5. The MRI contrast agent according to one of the preceding claims, **characterised in that** it additionally contains bentonite.

6. The MRI contrast agent according to claim 5, **characterised in that** it contains 25-40% by weight of kaolin and 4-5% by weight of bentonite.

7. The MRI contrast agent according to one of the preceding claims, **characterised in that** the kaolin, and where appropriate also the bentonite, have a particle size of 2.5-5 µm.

8. A process for producing an THE MRI contrast agent according to one of claims 1 to 4, **characterised in that** it comprises the following steps:
- dissolving citric acid and a citrate salt and, where appropriate, other excipients in 75-85% of the required water with stirring to form a citrate-buffered solution with a pH between 6 and 8,
- adding the kaolin in portions with stirring, in each case followed by stirring for several minutes to suspend the kaolin,
- adding the remaining water,
- finally stirring for several hours.

9. The process for producing an THE MRI contrast agent according to claim 5 or 6, **characterised in that** it comprises the following steps:
- dissolving citric acid and a citrate salt and, where appropriate, other excipients in 75-85% of the required water with stirring to form a citrate-buffered solution with a pH between 6 and 8,
- adding 70-85% of the kaolin in portions with stirring, in each case followed by stirring for several minutes to suspend the kaolin,
- mixing the remaining 30-15% of the kaolin with the bentonite in dry state to give a homogeneous mixture,
- adding the homogeneous mixture in portions to the previously prepared kaolin suspension with stirring, in each case followed by stirring for several minutes to suspend the homogeneous mixture,
- adding the remaining water,
- finally stirring for several hours.

10. The process according to either of claims 8 or 9, **characterised in that** the stirring to suspend the kaolin light and/or the homogeneous mixture takes place at 350 ± 50 rpm.

11. The process according to claim 10, **characterised in that** the stirring after each individual addition of kaolin or homogeneous mixture takes place for 10 to 15 min.

12. The process according to one of claims 8 to 11, **characterised in that** the final stirring for several hours takes place at 350 ± 50 rpm for at least 2 h.

13. The process according to one of claims 8 to 12, **characterised in that** the kaolin and, where appropriate, the bentonite are subjected beforehand to a micronization treatment to give a smaller particle size.

14. The process according to one of claims 8 to 13, **characterised in that** the THE MRI contrast agent is additionally stored after the final stirring.

15. The process according to claim 14, **characterised in that** the storage takes place for at least 24 h.

16. The process according to claim 15, **characterised in that** the storage takes place for 2 to 5 days.

## Revendications

1. Substance de contraste destinée à la RMN (tomographie par résonance magnétique) présentant une viscosité de 500 à 2500 cP, comprenant une suspension de 25-50 % en poids de kaolin dans 6-10 mmol/l solution tampon de citrate, pH 6-8.

2. Substance de contraste pour RMN selon la revendication 1, **caractérisée en ce que** le kaolin est mis en suspension dans une 7,5-8,2 mmol/l solution tampon de citrate, pH 6-8.

3. Substance de contraste pour RMN selon la revendication 1 ou la revendication 2, **caractérisée en ce que** le pH de la solution tampon de citrate se situe entre 6,5 et 7,5.

4. Substance de contraste pour RMN selon l'une quelconque des revendications 1 à 3, **caractérisée en ce qu'**elle contient de 25-40 % en poids de kaolin.

5. Substance de contraste pour RMN selon l'une quelconque des revendications précédentes, **caractérisée en ce qu'**elle contient en outre de la bentonite.

6. Substance de contraste pour RMN selon la revendication 5, **caractérisée en ce qu'**elle contient 25-40 % en poids de kaolin et 4-5 % en poids de bentonite.

7. Substance de contraste pour RMN selon l'une quelconque des revendications précédentes, **caractérisé en ce que** le kaolin et éventuellement aussi la bentonite présentent une grosseur de grain de 2,5-5 µm.

8. Procédé de fabrication d'une substance de contraste pour RMN selon l'une quelconque des revendications 1 à 4, **caractérisé en ce qu'**il comprend les opérations suivantes :
- dissoudre de l'acide citrique et un sel de citrate et éventuellement d'autre agents accessoires dans 75-85 % de l'eau nécessaire en agitant pour former une solution tampon de citrate présentant un pH compris entre 6 et 8,
- ajouter par portions le kaolin en agitant, opération suivie chaque fois d'une post-agitation de plusieurs minutes pour obtenir la suspension du kaolin,
- ajouter le reste de l'eau,
- agiter pour finir pendant plusieurs heures.

9. Procédé de fabrication d'une substance de contraste pour RMN selon la revendication 5 ou 6, **caractérisé en ce qu'**il comprend les opérations suivantes :
- dissoudre de l'acide citrique et un sel de citrate et éventuellement d'autres agents accessoires dans 75-85 % de l'eau nécessaire en agitant pour former une solution tamponnée de citrate présentant un pH compris entre 6 et 8,
- ajouter par portions de 70 - 85 % du kaolin en agitant, opération suivie chaque fois d'une post-agitation de plusieurs minutes pour obtenir la suspension du kaolin,
- mélanger le reste de 30 - 15 % du kaolin avec la bentonite à l'état sec en un mélange homogène,
- ajouter par portions le mélange homogène à la suspension de kaolin déjà fabriquée en agitant, opération suivie chaque fois d'une post-agitation de plusieurs minutes pour réaliser la suspension du mélange homogène,
- ajouter l'eau restante,
- agiter pour finir pendant plusieurs heures.

10. Procédé selon l'une des revendications 8 ou 9, **caractérisé en ce que** l'agitation pour réaliser la suspension du kaolin a lieu de façon modérée et / ou du mélange homogène est effectué à 350 ± 50 Upm.

11. Procédé selon la revendication 10, **caractérisé en ce que** la post-agitation a lieu après chaque addition individuelle de kaolin ou de mélange homogène pendant 10 à 15 min.

12. Procédé selon l'une quelconque des revendications 8 à 11, **caractérisé en ce que** l'agitation finale de plusieurs heures a lieu à 350 ± 50 Upm pendant au moins 2 h.

13. Procédé selon l'une quelconque des revendications 8 à 12, **caractérisé en ce que** le kaolin et éventuellement la bentonite sont soumis d'abord à un traitement de microminisisation à une plus faible grosseur de grain.

14. Procédé selon l'une quelconque des revendications 8 à 13, **caractérisé en ce qu'**on entrepose de plus la substance de contraste pour RMN après l'agitation finale.

15. Procédé selon la revendication 14, **caractérisé en ce que** l'entreposage a lieu pendant au moins 24 h.

16. Procédé selon la revendication 15, **caractérisé en ce que** l'entreposage a lieu pendant 2 à 5 jours.
